# EUROPEAN PATENT APPLICATION

(11) **EP 2 351 597 A1**
(43) Date of publication of application: **03.08.2011**
(21) Application number: 11151092.1
(22) Date of filing: 17.01.2011
(51) Int. Cl.: A61N 1/32

(54) **Portable apparatus for electro-stimulation of the human body**

(30) Priority: 15.01.2010 IT BO20100019
(71) Applicant: Group Talamonti di Talamonti Alessandro Impresa Induviduale, Acquaviva Picena (IT)
(72) Inventor: Talamonti, Alessandro, 63030 Acquaviva (IT)
(74) Representative: Jorio, Paolo

(57) **Abstract**

A portable apparatus for electro-stimulation of the human body, said apparatus (1) having a signal generator (29) for generating electrical signals having waveforms suited for electro-stimulation of a part of the human body, a pair of electrodes (3; 31; 32) for applying the electrical signals to the human body, a reservoir (4) for containing conductive gel, a pump (5) hydraulically connected to the reservoir (4) for delivering the conductive gel, and an outer shell (6) made of plastic material having a main body (7) and a head (8) that can be removed from a position of coupling to the main body (7). The main body (7) houses the signal generator (29) and the pump (5). The reservoir (4) can be removed from a position of coupling to the main body (7). The head (8) has a central portion (9), mounted on which is the pair of electrodes (3; 31; 32) and which is set, in use, in contact with the human body, and a hole (10) in the central portion (9) hydraulically coupled to the pump (5) for delivering the conductive gel outside the head (8).

## Description

The present invention relates to an apparatus for electro-stimulation of the human body, and in particular to a portable apparatus for electro-stimulation of the human body based upon the physical principle of molecular bioresonance.

An apparatus for electro-stimulation of the human body is known, which functions according to the principle of molecular bioresonance for the treatment of pathological conditions of traumatic, oedematous, circulatory origin and pathological conditions of the veno-lymphatic system. Said apparatus generates, in use, electrical signals obtained by superimposition of a first waveform oscillating at a first frequency on a second waveform oscillating at a second frequency higher than the first frequency. These electrical signals are applied, in use, to a part of the body of a patient via at least one pair of electrodes or conductive plates. The first frequency and the second frequency are parameterized with different values so as to create a plurality of elementary waveforms that are variously combined for generating a number of treatments.

In particular, said apparatus comprises a memory for storing data and parameters regarding the waveforms of each treatment, an electrical-signal generator for generating electrical signals according to the waveforms of the treatments, a microprocessor for controlling operation of the electrical-signal generator, interface means for enabling an operator to enter commands and monitor visually all the functional steps of the apparatus, and at least one pair of electrodes connected to the electrical-signal generator for application of the electrical signals to the body of the patient. The interface means comprise a keyboard for imparting commands to the microprocessor and a display device for enabling the operator to monitor visually all the functional steps of the apparatus. Furthermore, the apparatus comprises other devices and sensors for measuring the arterial pressure of the patient before, during, and after a given treatment and the impedance of the body of the patient, and a plurality of reservoirs, each coupled to a respective pump for delivering various types of conductive gel to be spread manually on the part of the body to be treated. Finally, the apparatus comprises a rechargeable lead battery for electrical supply of all the electronic devices of the apparatus itself.

The apparatus described above has been disclosed, in part and in terms of simplified block diagram, in the European patent application No. EP-A1-1163927.

Said apparatus is somewhat cumbersome on account of the various devices with which it is equipped, such as, for example, the keyboard for issuing commands, the display device, the reservoirs for the conductive gel, and the lead battery. Furthermore, the apparatus functions properly and effectively only if is used by authorized personnel to carry out the medical treatment on the patient, i.e., by skilled staff who are able to select the right treatment for the pathological condition to be treated and are able to position the electrodes correctly on the part of the body to be treated.

In other words, the apparatus has technical characteristics such as to render it particularly suited for a professional use, i.e., in a clinic, or else in a medical surgery or studio, and hence far from suited for domestic use for enabling the patient to continue the treatment privately even without the aid of skilled staff.

Moreover known are electro-stimulation apparatuses of a portable type, which comprise means for generation of electrical signals supplied by one or more batteries, but which, however, do not present the characteristics described in the European patent application No. EP-A1-1163927, i.e., they do not enable treatments to be conducted according to the principle of molecular bioresonance.

The aim of the present invention is to provide a portable apparatus for electro-stimulation of the human body that will be free from the drawbacks described above and, at the same time, will be easy and inexpensive to produce.

Provided in accordance with the present invention is a portable apparatus for electro-stimulation of the human body and the use of a portable apparatus for electro-stimulation of the human body for muscular rehabilitation therapies according to what is defined in the annexed claims.

The present invention will now be described with reference to the annexed drawings, which illustrate a non-limiting example of embodiment thereof and in which:
- Figure 1 is a side elevation of the portable apparatus for electro-stimulation of the human body provided according to the dictates of the invention;
- Figure 2 illustrates the apparatus of Figure 1 according to a perspective view from beneath;
- Figure 3 illustrates the apparatus of Figure 1 according to a perspective view from above;
- Figure 4 illustrates a simplified block diagram of the apparatus of Figure 1;
- Figure 5 illustrates an example of waveform generated by the apparatus of Figure 1;
- Figures 6 to 9 are views from beneath of different embodiments of a component of the apparatus of Figure 1; and
- Figure 10 illustrates, in greater detail and according to a view in longitudinal section, a further component of the apparatus of Figure 1.

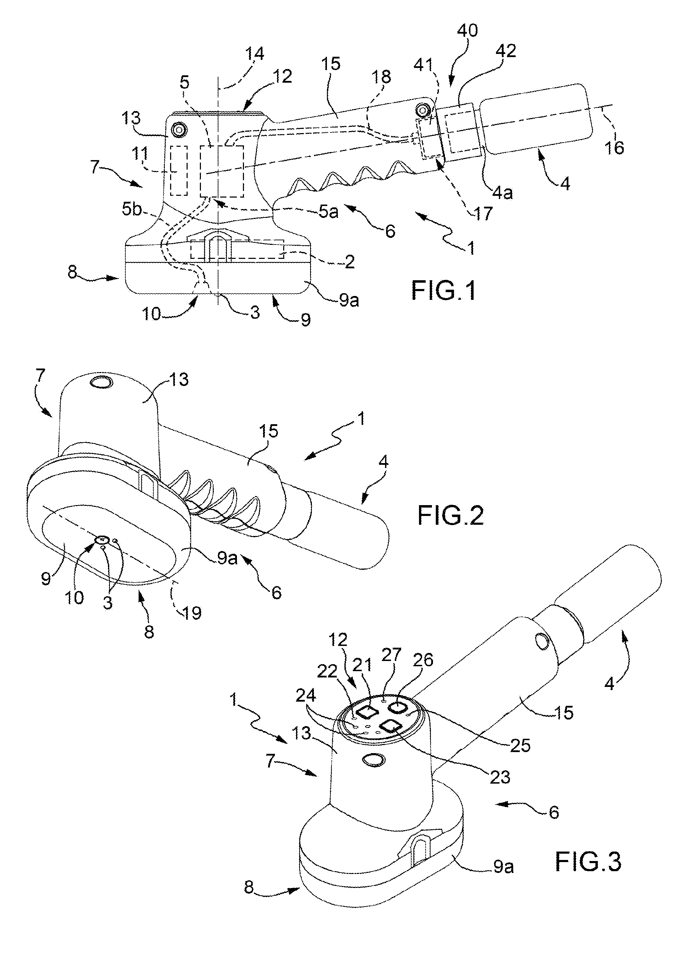

Designated as a whole by 1 in Figure 1 is the portable apparatus for electro-stimulation of the human body according to the present invention. The apparatus 1 comprises electronic signal-generating means 2 for generating electrical signals having given waveforms suited for electro-stimulation of a part of the human body, at least one pair of electrodes 3 designed to apply said electrical signals to the part of the human body, a reservoir 4 for containing conductive gel, a pump 5 hydraulically connected to the reservoir 4 for delivering, in use, the conductive gel, and an outer shell 6 made of plastic material, said outer shell 6 comprising a main body 7 and a head 8 which is removable from a position of coupling to the main body 7. Figure 1 illustrates the head 8 in the position of coupling to the main body 7.

The main body 7 houses within it the electronic signal-generating means 2 and the pump 5. The reservoir 4 is external to the main body 7 and is removable from a position of coupling to the main body 7. The head 8 has a central portion 9 (Figure 2), which is designed to be set in contact with the part of the human body, and a through hole 10 made in the central portion 9 and hydraulically coupled to an outlet 5a of the pump 5 via a hydraulic connection 5b for delivering the conductive gel outside the head 8. The pair of electrodes 3 is mounted on the central portion 9 of the head 8 so as to be, in use, in contact with the part of the human body. The head 8 is shaped like a lid. The central portion 9 of the head 5 is plane. Furthermore, the head 8 comprises a curved annular portion 9a that surrounds the central portion 9. The annular portion 9a has a surface radiused to the surface of the central portion 9 to favour sliding of the head 8 over the skin.

The apparatus 1 comprises a battery 11 for electrical supply of the electronic signal-generating means 2. The battery 11 is advantageously of a lithium-polymer rechargeable type. For example, the battery 11 has a capacity (electric charge) of 1850 mAh and a voltage Va of 7.4 V. The battery 11 is housed inside the main body 7.

The apparatus 1 comprises interface means 12 for enabling a user to send a command to the apparatus 1, i.e., for enabling a user to start or stop the pump 5 and to send commands to the electronic signal-generating means 2. The interface means 12 are mounted on the main body 7 so as to be readily accessible from outside by a user.

Once again with reference to Figure 1, the main body 7 comprises a cuplike portion 13, which has a symmetry with respect to an axis 14 and houses within it at least the pump 5 and the battery 11, and an oblong portion 15, which is fixed with respect to the cuplike portion 13, extends from the main body 7 along an axis 16 transverse to the axis 14, and is shaped so as to be easily gripped by a user's hand, for enabling him to use the apparatus 1 with just one hand. The oblong portion 15 has, at its free end, i.e., the end not connected to the cup-shaped body 13, a mouth 17 having a substantially cylindrical shape and hydraulically connected to the pump 5 via a tube 18 of small diameter. The reservoir 4 is substantially cylindrical and comprises a mouth 4a, which is cylindrical and is coaxial with the body of the reservoir 4.

The apparatus 1 further comprises a connector 40 for disposing, in a removable way, the reservoir 4 in the position of coupling to the main body 7. In said coupling position, the mouth 4a of the reservoir is hydraulically connected to the mouth 17, and the reservoir 4 is arranged parallel to the axis 16. In particular, the connector 40 has a first portion 41 that can be coupled in a fluid-tight way to the mouth 17, and a second portion 42 that can be coupled in a fluid-tight way to the mouth 4a of the reservoir 4. Furthermore, the connector 40 presents a circular symmetry such that the reservoir 4 arranges itself, when it is in the position of coupling to the main body 7, parallel to the axis 16. In Figure 1, the reservoir 4 is illustrated in the position of coupling to the main body 7. Advantageously from the ergonomic standpoint, the axis 16 intersects the axis 14 forming an angle of a value comprised between 82° and 77°. Preferably, the axis 16 intersects the axis 14 forming an angle of 80°.

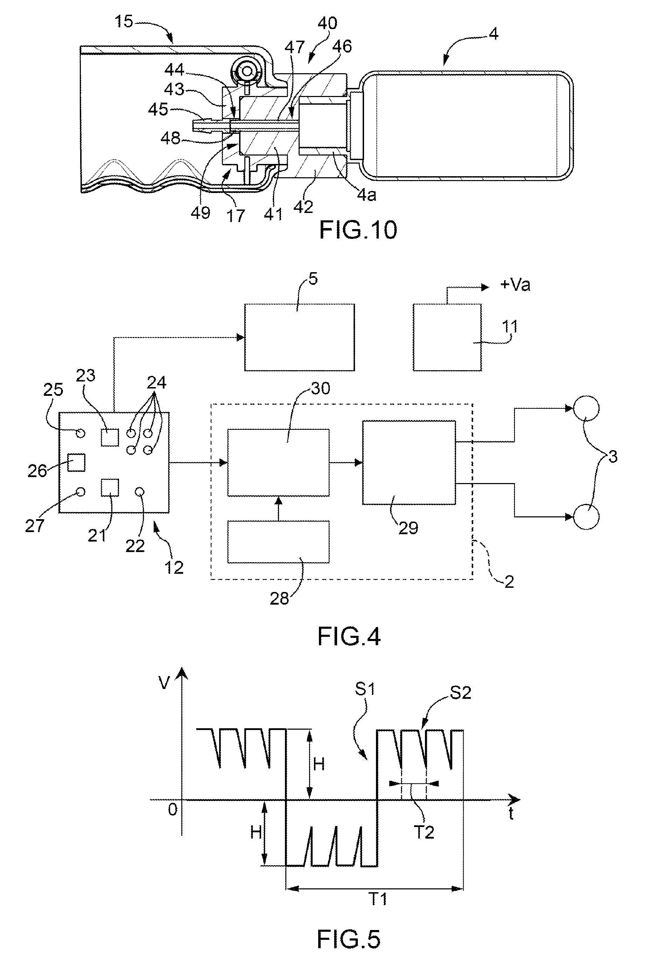

With reference to Figure 10, which illustrates the connection between the oblong portion 15, the connector 40, and the reservoir 4 in greater detail and according to a cross-sectional view along a plane passing through the axis 16, the mouth 17 comprises a bottom wall 43, in which a central hole 44 is made that is at least partially threaded, and a coupling element 45, which has a through hole communicating with the central hole 44 and is designed to fit in a fluid-tight way into the tube 18 (not illustrated in Figure 10). The portion 42 of the connector 40 is shaped like a cylindrical cup and is internally threaded. The mouth 4a of the reservoir 4 is externally threaded for being screwed into the portion 42. The portion 41 of the connector 40 comprises a longitudinal through hole 46 and a hollow tubular element 47, which is inserted and fixed in the through hole 46 and has a length such that an end portion 48 thereof projects from an external bottom surface 49 of the portion 41. The end portion 48 is externally threaded for being screwed into the central hole 44 so as to set the coupling element 45 in communication with the portion 42. The bottom wall 43 and the bottom surface 49 are plane and come into mutual contact when the tubular element 47 is completely screwed in the central hole 44. The connector 40 hence provides a hydraulic plug connection between the mouth 4a and the mouth 17, the plug being constituted by the tubular element 47.

In use, the conductive gel flows from the reservoir 4 to the pump 5 traversing the mouth 4a, the tubular element 47, the coupling element 45 and the tube 18. Furthermore, after the reservoir 4 has been filled up or replaced, it can be remounted on the main body 7 by first screwing the mouth 4a of the reservoir 4 in the portion 42 of the connector 40 and then inserting the portion 41 of the connector 40 in the mouth 17 and turning the connector 40 so that the tubular element 47 is screwed into the central hole 44 of the mouth 17. In this way, it is not necessary to keep the mouth 4a facing upwards to prevent the conductive gel from coming out of the reservoir 4 during the operation of fitting of the reservoir 4 in the main body 7.

According to a further embodiment of the invention (not illustrated), the mouth 4a and the portion 42 are not threaded, and the mouth 4a can be fitted in a fluid-tight way in the portion 42 in such a way that the reservoir 4 arranges itself parallel to the axis 16.

The plastic material of which the various parts of the outer shell 6, such as the main body 7 and the head 8, are made is of a type suited for medical use. Advantageously, the main body 7 is made of ABS (acrylonitrile butadiene styrene). The head 8 is made of polyurefin. Alternatively, the head 8 is made of makrolon, or else of PSU (polysulphone), or else of PPSU (polyphenylsulphone). The materials makrolon, PSU, and PPSU are resistant to treatments in autoclave, for example to a sterilization carried out in autoclave. The electrodes 3 are made of an electrically conductive material suited for medical use. Advantageously, the electrodes 3 are made of stainless steel. In particular, the electrodes 3 are made of so-called "stainless steel 304".

With reference to Figure 2, which is a perspective view from beneath of the apparatus 1, the central portion 9 has a longitudinal symmetry with respect to an axis 19, and the hole 10 has the centre that lies on the axis 19. The two electrodes 3 are arranged symmetrically with respect to the axis 19, i.e., on opposite sides with respect to the axis 19 and equidistant from the axis 19. The electrodes 3 have a circular shape. The central portion 9 has an area of between 5 cm² and 25 cm². The diameter of the electrodes 3 is relatively small as compared to the area of the central portion 9.

With reference to Figure 3, which is a perspective view from above of the apparatus 1, the interface means 12 are mounted on a bottom wall 20 of the cuplike portion 13 of the outer shell 6 so as to be readily accessible from outside by a user. The interface means 12 comprise a pushbutton 21 for starting and stopping the pump 5. The interface means 12 comprise a LED 22 for displaying the state of operation of the pump 5, i.e., for displaying whether the pump 5 is on (LED 22 on) or off (LED 22 off).

Furthermore, the interface means 12 comprise at least one pushbutton 23 for selecting one of a number of combinations of waveforms that can be generated by the electronic signal-generating means 2, each combination enabling execution, in use, of a respective treatment or therapy. The interface means 12 comprise a plurality of LEDs 24, each associated to a respective treatment for displaying which treatment has been selected. The interface means 12 further comprise a LED 25 for indicating the state of charge of the battery 11. In particular, the LED 25 turns on when the level of charge of the battery is low. Finally, the interface means 12 comprise a pushbutton 26 for switching on and switching off the apparatus 1 and a LED 27 to indicate the state (ON or OFF) of the apparatus 1.

With reference to Figure 4, which illustrates a simplified block diagram of the apparatus 1, the electronic signal-generating means 2 are substantially of the type described by the European patent application No. EP-Al-1163927. In particular, the electronic signal-generating means 2 comprise: a non-volatile memory 28 for storing data regarding a plurality of combinations of elementary waveforms, each of which combinations enables execution, in use, i.e., during generation of the corresponding waveforms, of a respective treatment or therapy; an electrical-signal generator module 29 for generating electrical signals according to the elementary waveforms; and a microprocessor 30 configured for controlling the electrical-signal generator module 29 in such a way that the latter will generate the electrical signals according to the combinations of elementary waveforms stored in the memory 28. Advantageously, the memory 28 stores at least two combinations of elementary waveforms. In the example of embodiment illustrated in Figures 3 and 4, the memory 28 stores four combinations of elementary waveforms. Hence, the LEDs 24 are four.

The electrodes 3 are connected at output of the electrical-signal generator module 29 for receiving the electrical signals generated. The microprocessor 30 is connected to the interface means 12 for receiving user commands. In particular, the microprocessor 30 is configured for selecting cyclically the combinations of elementary waveforms stored in the memory 28 whenever the pushbutton 23 is pressed. Furthermore, the microprocessor 30 is configured for confirming the combination selected when a given timeout that started after the last time the pushbutton 23 was pressed has been reached. Said technique of selection via multiple pressing of a pushbutton is in itself substantially known and is hence not described in further detail.

Figure 5 illustrates an example of the elementary waveforms used for obtaining the aforesaid combinations of waveforms. Each elementary waveform is a waveform obtained by superimposing a periodic waveform S1 with period T1 on a periodic waveform S2 with period T2 shorter than the period T1. In other words, each elementary waveform is a waveform obtained by superimposing the waveform S1 having frequency F1 on the waveform S2 having frequency F2 higher than the frequency F1. Advantageously, the frequency F1 is comprised between 0.3 and 7 Hz. Advantageously, the frequency F2 is comprised between 300 and 2300 Hz. Advantageously, as illustrated by the example of Figure 5, the waveform S1 is a square wave, and the waveform S2 is a triangular wave.

The waveforms S1 and S2 are voltage waveforms so as to obtain a voltage elementary waveform. The waveforms S1 and S2 are such that the elementary waveform oscillates between a positive value and a negative value symmetrical with respect to the voltage of 0 V. In other words, the waveforms S1 and S2 are such that the elementary waveform oscillates symmetrically around the voltage of 0 V with an amplitude H comprised between 7 and 20 V. Advantageously, the amplitude H is 12 V. The electrical-signal generator module 29 comprises a current limiter in itself known, and hence not illustrated, designed to limit, in use, the current supplied by the electrodes 3 to a maximum value comprised between 0.5 and 15 mA. Advantageously, the current supplied by the electrodes is limited to a maximum value of 10.5 mA. In other words, the apparatus 1 is designed to issue electrical signals constituted by low-voltage and low-frequency micro-currents.

By assigning different values to the frequencies F1 and F2 within the aforesaid frequency ranges it is possible to obtain a set of elementary waveforms. Each combination of elementary waveforms stored in the memory 28 comprises a sequence of some or all the elementary waveforms of the aforesaid set. Furthermore, associated to each elementary waveform of a certain combination is a respective generation time and a respective number of repetitions according to which, in use, generation of the elementary waveform is to be repeated. Each combination of elementary waveforms thus defined hence enables execution of a respective treatment.

In use, the user presses the pushbutton 26 for switching on the apparatus 1 and next, by gripping the apparatus 1 by the oblong portion 15, brings the central portion 9 of the head 8 into contact with the part of the human body that is to undergo treatment.

At this point, the user selects the desired combination of elementary waveforms, i.e., the treatment to be carried out, by pressing a certain number of times, in fast succession, the pushbutton 23. Pressing of the pushbutton 23 must be carried out in fast succession to prevent timeout before selection of the desired treatment. For example, on the hypothesis where the memory 26 stores four combinations (four treatments), pressing of the pushbutton 23 twice enables selection of the second combination, or else pressing of the pushbutton 23 five times enables selection of the first combination.

Once the combination selected is confirmed, the microcontroller 30 issues a command for execution of the corresponding treatment; i.e., it governs the electrical-signal generator module 29 so that it will generate electrical signals in accordance with the combination of elementary waveforms selected. In particular, each of the elementary waveforms of the combination selected is generated for the respective generation time and repeated for the respective number of repetitions. Furthermore, the various repetitions are spaced apart from one another by a period of pause of given duration, during which no electrical signal is issued.

It should be emphasized that, in use, the user can operate the pump 5 as desired by keeping the pushbutton 21 depressed for delivering the conductive gel before and/or during the treatment in such a way that the conductive gel, coming out of the hole 10, can be spread over the part of the human body undergoing treatment when the head 8 is in contact with the part of the body.

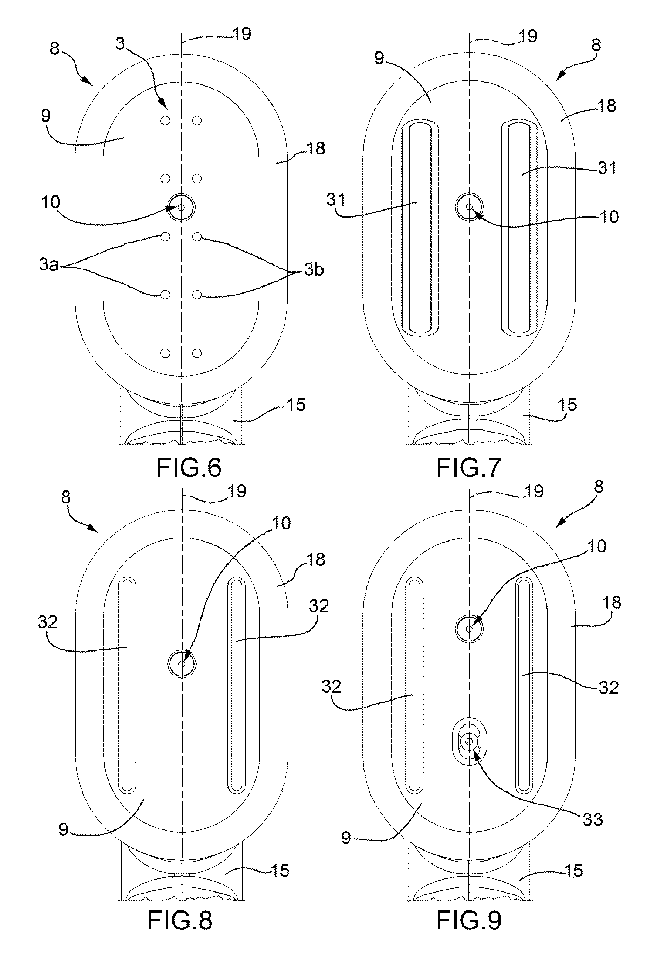

According to a further embodiment of the invention illustrated in Figure 6, where the head 8 is basically shown according to a view from beneath and where corresponding elements are designated by the same references as the ones used in Figure 2, a plurality of electrodes 3 are mounted on the central portion 9 of the head 8 and arranged in two parallel rows. Advantageously, the electrodes 3 of Figure 6 have the same shape (circular) as the electrodes 3 of Figure 2.

With reference to Figure 6, the electrodes of a first row are designated by 3a and those of the other row are designated by 3b. In particular, the two rows of electrodes 3a and 3b are arranged symmetrically with respect to the axis 19, i.e., on opposite sides with respect to the axis 19 and equidistant from the axis 19. Advantageously, the number of electrodes 3a is equal to the number of electrodes 3b in such a way that each electrode 3a of the first row is associated to a respective electrode 3b of the other row to form a corresponding pair of electrodes 3a, 3b. In other words, the apparatus 1 according to the embodiment of Figure 6 comprises a plurality of pairs of electrodes 3a, 3b. Advantageously, the electrodes of each row of electrodes 3a and 3b are electrically connected in parallel to one another. In this way, each pair of electrodes 3a, 3b receives one and the same electrical signal from the electrical-signal generator module 29.

The embodiment illustrated in Figure 6 is particularly advantageous in treatments in which there is the need to get the electrical signal to penetrate in depth in the part of the body human given the same intensity of the electro-stimulation. This is made possible by the substantially point-like shape of the electrodes 3.

According to a further embodiment of the invention illustrated in Figure 7, which basically shows the head 8 according to a view from beneath and where corresponding elements are designated by the same references as those used in Figure 6, the apparatus 1 comprises, instead of the electrodes 3 of Figure 2, at least one pair of electrodes 31 that have a substantially rectangular shape, rounded at the vertices of the rectangle, and are arranged at the same distances apart. In particular, the two electrodes 31 are mounted on the central portion 9 of the head 8 so as to be arranged symmetrically with respect to the axis 19, i.e., on opposite sides with respect to the axis 19 and equidistant from the axis 19. The electrodes 31 are made of the same material as the electrodes 3 of Figure 2.

The embodiment illustrated in Figure 7 favours passage of the electrical charges over wide areas of the skin of the part of human body undergoing treatment. For example, the head 8 according to the embodiment illustrated by Figure 7 is particularly indicated for treatments on pressure sores and sores in general. Advantageously, the hole 10 is arranged approximately half way along the electrodes 31. In this way, supply of the conductive gel from the hole 10 has the same effect irrespective of the direction of mutual displacement between the head 8 and the part of the human body along the axis 19.

According to a further embodiment of the invention illustrated in Figure 8, which basically shows the head 8 according to a view from beneath and where corresponding elements are designated by the same references as the ones used in Figure 7, the apparatus 1 comprises, instead of the electrodes 31 of Figure 7, at least one pair of electrodes 32 that have a substantially elongated rectangular shape, rounded at the vertices of the rectangle. By "elongated rectangular shape" is meant a rectangle in which the short side is shorter than or equal to 10% of the long side. In other words, the electrodes 32 of Figure 8 have a substantially rectilinear linear shape and are parallel to one another. Advantageously, the hole 10 is arranged approximately half way along the electrodes 32 for the same reason mentioned previously as regards the embodiment of Figure 7.

The embodiment illustrated in Figure 8 favours electroporation, i.e., a more effective absorption, through the skin of the part of human body undergoing treatment, of active principles possibly contained in the conductive gel. For this purpose it is of fundamental importance that the hole 10 for delivery of the conductive gel with the active principles should be arranged between the electrodes 32 approximately half way along the electrodes 32.

It should be emphasized that in all the embodiments of the head 8 illustrated in Figures 2, 6, 7 and 8, each electrode 3, 30, 31 or 32 has an area of between 0.5 and 3 cm². Said range of values of area is in proportion to the range of values of area of the central portion 9 of the head 8 mentioned previously.

According to a further embodiment of the invention illustrated in Figure 9, which basically shows the head 8 according to a view from beneath and where the corresponding elements are designated by the same references as the ones used in Figure 8, the head 8 comprises at least one further through hole 33 made in the central portion 9, and the apparatus comprises at least one further pump (not illustrated) having an inlet hydraulically connected to the hole 33 for generating, in use, a negative pressure on the part of the human body undergoing treatment when the head 8 is set in contact with the part of the human body. Advantageously, the hole 33 has the centre lying on the axis 19 (Figure 9). Furthermore, the centres of the holes 10 and 33 are arranged symmetrically with respect to the point half way along the electrodes 32. The distance between the centres of the holes 10 and 33 is such as to reduce to a minimum the risk of the hole 33 drawing in the conductive gel that has just been delivered from the hole 10.

It should be noted that the hole 33 and the particular arrangement between the hole 10 and the hole 33 can be combined also with the electrodes 3 and 31 of the variants illustrated in Figures 6 and 7, respectively.

The main body 7 houses inside it the further pump. In particular, the cuplike portion 13 houses inside it the further pump. Furthermore, the apparatus 1 comprises at least one further reservoir (not illustrated) hydraulically connected to an outlet of the further pump for collecting, in use, residue that may be drawn in by the further pump. The main body 7 houses within it, in a removable way, the further reservoir. In particular, the cuplike portion 13 houses, in a removable way, the further reservoir. Finally, the interface means 12 comprise a further pushbutton (not illustrated) for starting and stopping the further pump.

In use, the further pump applies, when operated by the user via the further pushbutton, a slight negative pressure on the skin of the part of the human body undergoing treatment, said negative pressure, in combination with a pressure of the head 8 on the skin exerted by the user, renders the treatment still more effective as regards movement of the liquids and stimulation of the connective system.

According to a further embodiment (not illustrated) of the invention, the apparatus 1 comprises a Peltier cell arranged in a point of the hydraulic connection 5b for modifying the temperature of the conductive gel being delivered as the treatment or therapy varies, i.e., for reducing or else raising the temperature of the conductive gel with respect to the room temperature as the treatment or therapy varies. In this way, it is possible to increase the effectiveness of each treatment or therapy.

It should be emphasized that the apparatus 1 described above can advantageously be used for the treatment of pathological conditions of traumatic, oedematous, circulatory origin and pathological conditions of the veno-lymphatic system. Furthermore, the apparatus 1 described above can be advantageously used for rehabilitation therapies. In particular, the head 8 of Figure 8 is the most suited for muscular rehabilitation in general. The head 8 of Figure 6 is the most suited for muscular rehabilitation when the muscle is "deep". The head 8 of Figure 7 is the most suited for rehabilitation therapies for ulcers, pressure sores, trophic lesions, diabetic foot, lymphoedema, oedema, edematous-fibro sclerotic panniculopathy (EFSP), and chronic venous insufficiency.

The main advantage of the portable apparatus 1 for electro-stimulation of the human body described above has an outer shell 6 with ergonomic shape that houses both the electronic means for generating electrical signals 2 and a pump 5 for delivery of conductive gel during the treatment. Furthermore, the particular arrangement of the reservoir 4 renders replacement or supply of the reservoir 4 itself simple and, at the same time, does not affect manageability of the apparatus 1. This renders the apparatus 1 suitable for a domestic use for enabling the patient to continue the treatment privately even without the aid of skilled staff.

Another advantage is represented by the removeable head 8, which facilitates cleaning thereof and enables replacement with another head 8 having a different number or type of electrodes (Figures 2, 6, 7, and 8). The interchangeability of the head 8 enables a portable apparatus 1 to be obtained that ensures a high effectiveness in the treatment of a number of pathological conditions or in the execution of rehabilitation therapies, given the same combinations of elementary waveforms implemented by the electronic signal-generating means 2. In other words, given a particular treatment to be carried out, it is possible to choose the most suitable head 8 (the electrodes) from among a number of heads 8 available.

Finally, the embodiment with the further pump and the further reservoir renders any treatment even more effective as regards movement of the liquids and stimulation of the connective system.

## Claims

1. A portable apparatus for electro-stimulation of the human body, the apparatus (1) comprising electronic signal-generating means (2) for generating electrical signals having given waveforms that are suited for electro-stimulation of a part of the human body, and at least one pair of electrodes (3; 31; 32) designed to apply said electrical signals to said part of the human body; and **being characterized in that** it comprises a first reservoir (4) for containing a conductive gel, a first pump (5) hydraulically connected to the first reservoir (4) for delivering, in use, the conductive gel, and an outer shell (6) made of plastic material comprising a main body (7) and a head (8) which is removable from a position of coupling to the main body (7); said main body (7) housing the electronic signal-generating means (2) and the first pump (5); said reservoir (4) being removable from a position of coupling to the main body (7); said head (8) having a central portion (9), mounted on which is said pair of electrodes (3; 31; 32) and which is designed to be set, in use, in contact with said part of the human body, and a first hole (10) made in the central portion (9) and hydraulically coupled to an outlet (5a) of the first pump (5) for delivering the conductive gel outside the head (8).

2. The apparatus according to Claim 1, wherein said main body (7) comprises a cuplike portion (13), which has a symmetry with respect to a first axis (14) and houses at least said first pump (5), and an oblong portion (15), which is fixed with respect to said cuplike portion (13), extends from said main body (7) along a second axis (16) transverse to the first axis (14) and is shaped so that it can be gripped by the hand of a user.

3. The apparatus according to Claim 2, wherein said oblong portion (15) comprises a first mouth (17) hydraulically connected to said first pump (5), and said reservoir (4) comprises a second mouth (4a); the apparatus (1) comprising connection means (40) for disposing, in a removable way, said reservoir (4) in said position of coupling to said main body (7), wherein said second mouth (4a) is hydraulically connected to said first mouth (17) and said reservoir (4) is arranged parallel to said second axis (16).

4. The apparatus according to any one of Claims 1 to 3, wherein said central portion (9) of said head (8) is plane.

5. The apparatus according to Claim 4, wherein said central portion (9) has an area of between 5 cm² and 25 cm².

6. The apparatus according to any one of Claims 1 to 5, wherein each electrode (3; 31; 32) of said at least one pair of electrodes has an area of between 0.5 cm² and 3 cm².

7. The apparatus according to any one of Claims 1 to 6, wherein the electrodes (31; 32) of said at least one pair of electrodes have a rectangular shape and are arranged at equal distances apart.

8. The apparatus according to any one of Claims 1 to 6, wherein the electrodes (32) of said at least one pair of electrodes have a rectilinear linear shape and are parallel to one another.

9. The apparatus according to any one of Claims 1 to 8, wherein said central portion (9) has a longitudinal symmetry with respect to a third axis (19); said first hole (10) having the centre lying on said third axis (3) and the electrodes (3; 31; 32) of said at least one pair of electrodes being arranged symmetrically with respect to said third axis (19).

10. The apparatus according to any one of Claims 1 to 6, wherein the electrodes (3) of said at least one pair of electrodes have a circular shape.

11. The apparatus according to Claim 10, wherein said at least one pair of electrodes comprises a plurality of electrodes (3) arranged in two parallel rows.

12. The apparatus according to any one of Claims 1 to 11, wherein said head (8) comprises a second hole (33) made through said central portion (9); the apparatus (1) further comprising at least one second pump having an inlet hydraulically connected to the second hole (33) for generating, in use, a negative pressure on said part of the human body when said head (8) is set in contact with said part of the human body; said main body (7) of said outer shell (6) housing said second pump.

13. The apparatus according to Claim 12, comprising a second reservoir hydraulically connected to an outlet of said second pump for collecting, in use, residue that may be drawn in by the second pump; said main body (7) housing the second reservoir in a removable way.

14. The apparatus according to any one of Claims 1 to 13, wherein said electronic signal-generating means (2) comprise a memory (28) for storing data regarding a plurality of combinations of elementary waveforms, each combination of elementary waveforms enabling execution, in use, of a respective treatment; the apparatus (1) comprising interface means (12) for enabling a user to send a command to the apparatus (1); the interface means (12) comprising: at least one pushbutton (23) for selecting the treatment to be carried out; and a plurality of LEDs (24), each associated to a respective treatment for displaying which treatment has been selected.

15. The apparatus according to Claim 14, wherein said electronic signal-generating means (2) comprise a microcontroller (30) configured for controlling generation of said electrical signals according to said combinations of elementary waveforms; the microcontroller (30) being configured for selecting in a cyclic way each of said combinations of elementary waveforms stored in the memory (28) whenever said pushbutton (23) is pressed.

16. The apparatus according to Claim 14 or Claim 15, wherein each said elementary waveform is obtained by superimposing a first waveform (S1) oscillating at a first frequency (F1) on a second waveform (S2) oscillating at a second frequency (F2) higher than the first frequency (F1).

17. The apparatus according to any one of Claims 1 to 16, comprising a hydraulic connection (5b) for coupling said outlet (5a) of said first pump (5) hydraulically to said first hole (10) and a Peltier cell arranged in a point of said hydraulic connection (5b) for modifying the temperature of said conductive gel being delivered.

18. Use of an apparatus for electro-stimulation of the human body for rehabilitation therapies, said apparatus (1) being of the type claimed in any one of Claims 1 to 17.
